(19) Europäisches Patentamt
European Patent Office
Office européen des brevets

(11) Numéro de publication: **0 324 671 A2**

# (12) DEMANDE DE BREVET EUROPEEN

(21) Numéro de dépôt: **89400050.4**

(22) Date de dépôt: **09.01.89**

(51) Int. Cl.$^4$: **C 07 C 91/02**
C 07 F 7/08
// C07F5/00

(30) Priorité: **14.01.88 US 144326 08.12.88 US 280541**

(43) Date de publication de la demande:
**19.07.89 Bulletin 89/29**

(84) Etats contractants désignés:
**AT BE CH DE FR GB IT LI NL SE**

(71) Demandeur: **RHONE-POULENC CHIMIE**
**25, quai Paul Doumer**
**F-92408 Courbevoie Cédex (FR)**

(72) Inventeur: **Gradeff, Peter S.**
**167 Black River Road**
**Pottersville New Jersey 07979 (US)**

**Mauermann, Heiko**
**38 Hartwell Street**
**New Brunswick New Jersey 08901 (US)**

**Yunlu, Kenan**
**50 Robinson Street**
**New Brunswick New Jersey 08901 (US)**

**Ramirez, Carlos M.**
**10 Wycoff Avenue**
**Piscataway New Jersey 08854 (US)**

(74) Mandataire: **Dutruc-Rosset, Marie-Claude et al**
**RHONE-POULENC INTERSERVICES Service Brevets**
**Chimie 25, Quai Paul Doumer**
**F-92408 Courbevoie Cédex (FR)**

## (54) Dérivés de type alcoxy des métaux trivalents du groupe 3b et leur procédé d'obtention.

(57) La présente invention a pour objet des dérivés de type alcoxy des métaux trivalents du groupe 3b, notamment cérium III tels que alcoxydes, silyloxides ou alcanolatoamines.

Ils sont préparés à partir d'un complexe anhydre des métaux trivalents du groupe 3b, par exemple, complexe nitrate cérique d'ammonium, en présence d'un alcool, d'un silanol ou d'une alcanolamine et une base ou en mettant en oeuvre le complexe et les sels alcalins de l'alcool, du silanol ou de l'alcanolamine.

EP 0 324 671 A2

## Description

## DERIVES DE TYPE ALCOXY DES METAUX TRIVALENTS DU GROUPE 3b ET LEUR PROCEDE D'OBTENTION

La présente invention concerne des dérivés de type alcoxy des métaux du groupe 3b incluant les métaux trivalents de terres rares, notamment le cérium, tels qu'alcoolates, silyloxydes ou composés alcanolatoamines et des procédés pour préparer ces composés.

Les dérivés alcoxy de métaux polyvalents sont des composés organométalliques à usages multiples. Les alcoolates, par exemple, ont été utilisés comme additifs de peintures, hydrofugeants, activeurs d'adhérence, mordants, agents d'apprêt dans les compositions d'émaux, catalyseurs, précurseurs de céramiques et de fibres, et également comme intermédiaires dans la synthèse d'autres composés organiques de métaux. Il existe quatre méthodes générales de préparation des alcoolates métalliques, toutes en conditions anhydres, qui sont les suivantes :

A. Par réaction de l'alcool correspondant et d'un métal tel que les métaux alcalins, les métaux alcalino-terreux et l'aluminium, à l'aide d'un catalyseur alcalin ou acide.

B. Par réaction de l'alcool correspondant avec les oxydes et hydroxydes des métaux, par exemple NaOH ou $Na_2O$, $V_2O_5$ et $MoO_3.2H_2O$.

C. Par réaction de l'alcool correspondant et de l'halogénure métallique en présence d'un base anhydre. Un exemple typique est la préparation de $Th(OR)_4$ ou $Zr(OR)_4$ :

$$ThCl_4 + 4ROH + 4NaOR \longrightarrow Th(OR)_4 + 4NaCl$$

$$ZrCl_4 + 4ROH + 4NH_3 \longrightarrow Zr(OR)_4 + 4NH_4Cl$$

La réaction peut être utilisée pour préparer des alcoolates de titane, d'hafnium, de germanium, de niobium, de tantale, d'aluminium et d'étain.

D. Par transéthérification des alcoolates métalliques d'alcools inférieurs, comme les méthylates, éthylates ou isopropylates, avec un alcool supérieur.

La méthode A est illustrée pour un certain nombre d'alcoolates d'yttrium, de lanthane et d'autres lanthanides par L. Brown et K. Mazdiyasni dans <u>Organic Chemistry</u>, (1970) page 2783. La réaction, que l'on ne croyait antérieurement utile que pour les métaux alcalins, le magnésium et l'aluminium, a été étendue par les auteurs à la synthèse des isopropylates d'yttrium et de tous les lanthanides. Pour les lanthanides inférieurs, tels que lanthane, cérium, praséodyme et néodyme, on utilise comme catalyseur un mélange de $HgCl_2$ et $Hg(C_2H_3O_2)_2$ ou $HgCl_2$ pour augmenter à la fois la vitesse de réaction et le rendement (en %). En général, on fait réagir 5 g de tournures du métal avec environ 300 ml d'alcool isopropylique à la température de reflux pendant environ 24 h et en présence d'une faible quantité du sel de Hg comme catalyseur. Les auteurs disent que les rendements sont de 75 % ou mieux.

La plupart des autres exemples, dans la littérature, de la préparation d'alcoolates de lanthanides concernent l'utilisation des halogénures métalliques correspondants. Dans certains cas, on préfère un complexe $LaCl_3$, 3ROH au chlorure $LaCl_3$ (Misra et autres, Austr. J. <u>Chem.</u> 21,page 797 (1978) et Mehrotra et Batwara, <u>Inorganic Chem.</u> 9, page 2505 (1970).

Une variante intéressante de la méthode D est mentionnée par Tripathi, Batwara et Mehrotra dans <u>J.C.S.A.</u> (1976), page 991. Les alcoolates inférieurs d'ytterbium (tels que méthylate et éthylate) ont été synthétisés à partir de l'isopropylate d'ytterbium par transéthérification par le méthanol ou l'éthanol. En raison de leur faible solubilité, ces alcools sont séparés par précipitation au fur et à mesure de la réaction, en déplaçant l'équilibre jusqu'à la transéthérification complète.

En général, les méthodes A, B, et C ne conviennent que pour la préparation des alcoolates inférieurs tels que les méthylates, éthylates et isopropylates, puisque la réactivité des alcools supérieurs diminue lorsque leurs poids moléculaires augmentent. Les alcoolates supérieurs sont mieux préparés par la méthode D qui est un procédé en deux étapes.

Les brevets des EUA 4 489 000 et 4 492 655 aux noms de Gradeff et Schreiber décrivent la préparation d'alcoolates de cérium trétravalent à partir du nitrate cérique d'ammonium (ou hexanitratocérate (IV) d'ammonium). Ces brevets sont incorporés par référence à la présente description.

La demande de brevet EUA Serial n° 895 560 également incorporée par référence, concerne des dérivés alcanolatoamines de cérium tétravalent et leur procédé de fabrication à partir de nitrate cérique d'ammonium. Le brevet EUA 4 663 439 de GRADEFF et SCHREIBER qui décrit un procédé de préparation d'alcoolates cériques, est également incorporé par référence.

Les techniques modernes de séparation pour les lanthanides donnent ces éléments principalement sous forme de chlorures ou nitrates en solution aqueuse. Ils sont utilisés pour fabriquer tous les autres dérivés tels que carbonates, hydroxydes, oxydes, etc. Ils peuvent aussi être utilisés pour fabriquer certains des dérivés organiques tels que les alkylcarboxylates supérieurs ou les acétylacétonates. Cependant, les sels inorganiques de lanthanides <u>anhydres</u>, sont essentiels pour fabriquer les alcoolates, les dérivés ayant des liaisons Ln-carbone et un grand nombre d'autres ainsi que pour la production de métaux par des procédés électrolytiques et métallothermiques. Les oxydes peuvent être fabriqués à l'état anhydre, mais leur faible solubilité et leur réactivité limitée excluent leur utilisation dans de nombreux domaines. Les halogénures sont la seule classe de composés qui sont utilisés comme source de composés anhydres convenant à cet effet. Les plus faciles à fabriquer sont les fluorures, mais ils n'ont qu'une utilisation limitée dans les synthèses en raison d'une solubilité et d'une réactivité extrêmement faibles. Les iodures sont parmi les plus difficiles à

déshydrater. Les bromures et chlorures présentent un degré semblable de difficulté qui est plus grand que pour les fluorures, mais moindre que pour les iodures. Au vu de considérations économiques et d'environnement, les chlorures sont les sels de lanthanides anhydres les plus recherchés. Ceci comprend la cérium qui est le plus abondant des lanthanides.

Les halogénures qui se séparent de leurs solutions aqueuses retiennent ordinairement 6 à 7 moles d'eau. Après séparation de l'eau non liée, la majeure partie de l'eau liée peut être séparée par déshydratation soignée au-dessous de 100°C. Il est cependant extrêmement difficile de séparer la dernière mole d'eau sans décomposer l'halogénure.

La déshydratation des hydrates de chlorure de lanthane peut être effectuée par HCl gazeux, mais c'est un procédé fastidieux. La déshydratation utilisant le chlorure d'ammonium en plus d'HCl et à des températures au-dessous d'environ 200-300°C a donné les meilleurs résultats. On a fait réagir des oxydes avec le monochlorure de soufre et le chlore ou le monochlorure de soufre seul. Le chlorure de thionyle, le tétrachlorure de carbone et le phosgène ont également été utilisés comme réactifs. La préparation du sel anhydre des lanthanides le plus couramment utilisé, le chlorure, n'est pas facile (Chem. Rev, 1962, pages 503-511).

Le brevet des EUA n° 4 492 655 aux noms de Gradeff et Schreiber décrit la préparation des dérivés cyclopentadiénylés du cérium (III) utilisant le nitrate cérique d'ammonium et le cyclopentadiényl-Na. Par l'utilisation d'un nitrate, on s'est écarté des voies classiques. Le nitrate cérique d'ammonium avait l'avantage d'être facilement obtenu sous forme anhydre. Le procédé de cette invention consiste à ajouter lentement le cyclopentadiénylure de métal alcalin à une solution de nitrate cérique d'ammonium pour former successivement le mono- jusqu'au tricyclopentadiényl-cérium. L'équation globale de la réaction est la suivante :

$2[Ce(NO_3)_4.\ 2NH_4NO_3] + 12NaCp \rightarrow 2Ce\ (Cp)_3 + 4CpH + (Cp)_2 + 12NaNO_3 + 4NH_3.$

On montre que l'étape de réduction "in situ" se déroule par deux voies possibles :

(a) $Ce(NO_3)_4 + 4NaCp \rightarrow [Ce(Cp)_4] + 4NaNO_3$

$[2Ce(Cp)_4] \rightarrow [2Ce(Cp)_3] + Cp_2$

(b) $Ce(NO_3)_6.\ 2NH_4 + NaCP \rightarrow [Ce^{+4}\ Cp(NO_3)_5.\ 2NH_4] + NaNO_3 \rightarrow Ce^{+3}\ (NO_3)_5.\ 2NH_4 + 1/2\ Cp.Cp$

La réaction "in situ" ci-dessus est intéressante dans les cas où le réactif est un bon agent réducteur, une partie du réactif étant consommée dans la réduction de $Ce^{4+}$ en $Ce^{3+}$. Dans les cas comme celui indiqué ci-dessus, ceci peut être assez coûteux.

Un des objets de la présente invention est de fournir des complexes de nitrates des métaux du groupe 3b trivalents incluant l'yttrium et les lanthanides tels que le cérium.

Un autre objet de la présente invention est de fournir des dérivés alcoxy des métaux trivalents du Groupe 3b incluant l'yttrium et les lanthanides tels que le cérium.

La présente invention est un procédé approprié pour fabriquer des dérivés alcoxy et nitrates anhydres des métaux du Groupe 3b (lanthanons) incluant l'yttrium et les lanthanides tels que le cérium. Il utilise un nouveau produit de départ et une nouvelle voie de synthèse évitant d'utiliser les halogénures de lanthanides trivalents, par exemple céreux, anhydres coûteux pour produire divers dérivés alcoxy, dont certains sont connus et d'autres sont des composés nouveaux.

Conformément à la présente invention, des dérivés alcoxy des métaux du Groupe 3b, par exemple, lanthanides, trivalents anhydres peuvent être préparés en faisant réagir, dans des conditions anhydres, un complexe anhydre nitrate de lanthanon-métal alcalin ou un complexe nitrate de lanthanon-nitrate d'ammonium avec un alcool, un silanol ou une alcanolamine appropriés tels que définis ci-après, en présence d'une base. Ces dérivés anhydres peuvent être également préparés en faisant réagir le complexe avec un sel de métal alcalin, de l'alcool, du silanol ou de l'alcanolamine qui correspond à l'intermédiaire formé in situ quand l'alcool, le silanol ou l'alcanolamine est en présence de la base susmentionnée.

Le complexe anhydre nitrate de lanthanon trivalent-métal alcalin ou nitrate d'ammonium peut être préparé selon divers procédés tels que mentionnés ci-après.

La présente invention a trait à la préparation de dérivés alcoxy de lanthanon et complexe nitrate anhydre. Par lanthanon ou lanthanide, on entend les métaux trivalents du Groupe 3b du Tableau périodique des éléments (excluant les actinides) mais incluant l'yttrium (numéro atomique 37) et les lanthanides de numéro atomique 57-71 et leurs mélanges.

Les membres plus abondants tels que l'yttrium, le lanthane, le cérium, le praséodyme et le néodyme sont particulièrement importants. Les terres rares les plus préférées sont l'yttrium, le lanthane et le cérium.

En référence au cérium, ces objets et d'autres peuvent être atteints en utilisant du nitrate cérique d'ammonium préalablement séché, composé de cérium (IV) disponible dans le commerce. Selon un procédé de la présente invention, on fait réagir le complexe anhydre nitrate céreux-nitrate d'ammonium formé par réaction du nitrate cérique d'ammoniun anhydre (CAN) avec un agent réducteur convenable dans des conditions anhydres, avec un alcool, un silanol, ou une alcanolamine représentés par HOX en présence d'une base et en l'absence d'eau pour produire des dérivés du type alcoxy du cérium trivalent anhydre.

Dans un mode de mise en oeuvre de l'invention (Procédé A), le CAN anhydre est traité par l'ammoniac dans des conditions anhydres pour obtenir le complexe nitrate céreux-nitrate d'ammonium anhydre que l'on fait ensuite réagir en l'absence d'eau avec un alcool, un silanol, ou une éthanolamine en présence d'une base.

Dans un autre mode de mise en oeuvre (Procédé B), le CAN sec est mis en contact avec un autre agent réducteur convenable jusqu'à réduction complète en $Ce^{3+}$.

On fait ensuite réagir le composé de $Ce^{3+}$ résultant avec l'alcool souhaité, le silanol ou l'alcanolamine (notamment l'éthanolamine) en présence d'une base, en l'absence d'humidité.

## PROCEDE A

Equation A-1 $3(NH_4)_2Ce(NO_3)_6 + 4NH_3 \rightarrow 3Ce(NO_3)_3 + 1/2N_2 + 9NH_4NO_3$

A-2 $Ce(NO_3)_3 + 3HOX + NH_3 \rightarrow Ce(OX)_3 + 3NH_4NO_3$

Le Procédé A consiste à soumettre le complexe hexanitratocérate IV d'ammonium anhydre séché (aussi appelé nitrate cérique d'ammonium ou CAN) à un traitement à l'ammoniac selon l'équation A-1, le $Ce^{4+}$ étant ainsi réduit en $Ce^{3+}$ en conditions anhydres. Le complexe résultant de nitrate de $Ce^{3+}$ et de nitrate d'ammonium est utilisé comme source de sel anhydre de $Ce^{3+}$. L'ammoniac est économique et donne un sous-produit simple, l'azote, en plus du nitrate d'ammonium.

Le nitrate cérique d'ammonium est un produit du commerce qui peut être débarrassé de toute son eau par simple séchage dans un four à 105-120° C sous pression atmosphérique pendant environ 6-12 h, ou sous vide à une température inférieure.

Selon la présente invention, le CAN séché soit à l'état solide, soit en suspension ou dissous dans un solvant inerte approprié, est mis en contact avec le gaz d'ammoniac.

Les tentatives pour isoler $Ce(NO_3)_3$ à l'état libre n'ont pas réussi. Apparemment l'ion $Ce^{3+}$ a besoin d'être encore lié par coordination. Le nitrate d'ammonium facilite la coordination et permet l'élimination de l'eau à des températures plus basses, jusqu'à l'état anhydre. Comme dans le cas du CAN et d'autres exemples courants pour les éléments lanthanides, les ligands coordinés peuvent être séparés dans des réactions ultérieures si on le désire et ne posent pas de problème majeur. Une certaine quantité de $NH_3$ est également liée au niveau moléculaire au complexe de nitrate de cérium et dans certains cas sa présence peut être souhaitable. Par exemple, lorsque le produit de $Ce^{3+}$ anhydre résultant doit être mis à réagir avec un alcool ou un silanol en présence de $NH_3$, le $NH_3$ coordiné constitue une portion du $NH_3$ nécessaire. Dans d'autres cas, sa présence peut ne pas être souhaitable et on doit le séparer, par exemple par pompage sous vide, chauffage, neutralisation par HCl sec ou déplacement par un autre agent de coordination.

Le traitement du CAN solide, préalablement séché, par $NH_3$ peut être effectué de diverses manières. Le contact du CAN avec $NH_3$ peut être maintenu pendant une durée suffisante pour réduire tout le $Ce^{4+}$ en $Ce^{3+}$. La durée nécessaire à cet effet dépend du mode de contact, de la granulométrie du CAN, de la température et de la pression. La mise en contact du CAN avec $NH_3$ peut être effectuée sous pression atmosphérique, par exemple, tout en agitant le CAN sous $NH_3$. L'ammoniac peut être maintenu sous pression ou utilisé sous forme liquide. Lorsque l'on utilise $NH_3$ liquide, l'excès est facilement récupéré après la fin de la réaction.

La température pendant le contact peut être aussi basse que la température de l'ammoniac liquide ou aussi élevée qu'environ 130° C, mais inférieure à la température de décomposition du CAN, qui commence à environ 170° C.

Le CAN peut être réduit en suspension ou en solution dans des solvants inertes tels que benzène, pentane, éthers de pétrole ou éthers méthyliques de glycols (notamment le diglyme). Il n'est pas nécessaire d'isoler le produit du solvant inerte.

Une fois que la réduction complète par $NH_3$ a été effectuée, on peut ajouter des réactifs pour effectuer la conversion subséquente du complexe nitrate de $Ce^{3+}$ anhydre en le nouveau dérivé désiré.

Le complexe de nitrate céreux anhydre soit solide, soit en suspension dans un solvant inerte, est mélangé avec un alcool, un silanol ou une alcanolamine. On ajoute ensuite une base telle que $NH_3$, métal alcalin ou bien l'alcool, le silanol ou l'alcanolamine sous forme de sel de métal alcalin.

Par mise en contact avec la base, on forme in situ un sel correspondant de l'alcool, du silanol ou de l'alcanolamine. Ainsi, une autre variante, est de mélanger le complexe avec le sel alcalin correspondant à l'alcool, au silanol ou à l'alcanolamine. Si l'alcool, le silanol ou l'alcanolamine est mélangé avec le sel alcalin du même alcool, silanol ou alcanolamine, l'alcool sert alors de solvant et la réaction a lieu entre le complexe et le sel. Si l'alcool, le silanol ou l'alcanolamine est mélangé avec un sel alcalin d'un alcool, silanol ou alcanolamine différents, il pourrait y avoir des échanges de ligands, de sorte que les ligands soit de l'alcool, du silanol ou de l'alcanolamine ou du sel pourraient réagir avec le complexe.

Le produit est normalement maintenu en solution en utilisant un solvant inerte approprié tel que le DME, d'autres éthers méthyliques de glycols, (notamment le diglyme), le tétrahydrofurane (THF), le benzène, le toluène, etc. Le solvant peut être séparé par filtration du nitrate d'ammonium ou de métal alcalin formé comme sous-produit.

## PROCEDE B

Bien que la réduction du CAN par $NH_3$ soit la manière la plus efficace et la moins coûteuse pour produire le complexe de nitrate de $Ce^{3+}$ souhaité, un grand nombre d'autres agents réducteurs conviennent pour l'invention. Les équations B-1 et B-2 illustrent le procédé :

B-1 $(NH_4)_2Ce(NO_3)_6$ + (ra) → $Ce(NO_3)_3,2NH_4NO_3$ + (PrOX) + $HNO_3$

B-2 $Ce(NO_3)_3,2NH_4NO_3$ + $HNO_3$ + (PrOX) + 3HOX + $4NH_3$ → $Ce(OX)_3$ + $6NH_4NO_3$ + (PrOX)

Le composé (ra) est un agent réducteur, qui peut être par exemple HOX, tel un dérivé destiné à fournir également le lignand alcoxy OX, de préférence un alcanol inférieur, le produit (PrOX) étant moins gênant et plus facile à séparer.

La caractéristique qui distingue ce procédé du brevet EUA 4 492 655 est que $Ce^{4+}$ doit être réduit en $Ce^{3+}$ avant la réaction. Si cette étape essentielle de réduction n'a pas lieu, les produits de la réaction globale résultants seront les dériv+és alcoxy de $Ce^{4+}$, comme décrits dans les brevets EUA 4 489 000, 4 492 655 et la demande EUA Serial n° 895 560, et non pas ceux de $Ce^{3+}$ désirés.

De nombreux agents réducteurs conviennent pour réduire $Ce^{4+}$ en $Ce^{3+}$ selon l'invention. Un exemple extrême est le diméthoxyéthane (DME), considéré comme un solvant inerte. Le DME réagit avec le CAN à la température de reflux pour réduire totalement $Ce^{4+}$ en $Ce^{3+}$. Les agents réducteurs les plus intéressants, cependant, à cause de leur bas prix, de leur facilité relative à effectuer la réaction et de leur facilité relative à séparer le produit d'oxydation (PrOX), si nécessaire, sont les alcanols inférieurs, ayant de préférence moins de 4 atomes de carbone, tels que méthanol, éthanol, propanol, isopropane, etc. En général, l'agent réducteur approprié pour la présente invention est un agent qui réagit comme réducteur en milieu anhydre et conduit à une réduction pratiquement complète de $Ce^{4+}$ en $Ce^{3+}$. La réduction peut se dérouler vite ou lentement, selon l'agent réducteur. Elle peut être accélérée, si on le désire, par exposition aux UV, chauffage ou addition d'un catalyseur approprié. Pendant cette étape du procédé, $Ce^{4+}$ est réduit en $Ce^{3+}$, tandis que le milieu rest anhydre. Comme indiqué dans l'équation B-1, de l'acide nitrique est produit pendant la réduction et reste tel quel dans le mélange si l'agent réducteur est un produit neutre comme un alcool. Outre l'acide nitrique, le mélange peut aussi contenir un sous-produit d'oxydation (PrOX). Dans la plupart des cas, l'acide nitrique et le (PrOX) peuvent être conservés pour la seconde étape qui consiste à faire réagir l'alcool, le silanol ou l'alcanolamine souhaité(e) avec le $Ce^{3+}$ réduit en présence d'une base qui neutralise également l'acide. Cependant, l'acide nitrique peut être neutralisé et le PrOX séparé du système avant la réaction avec HOX.

Selon une autre variante de la présente invention, des complexes nitrate de métal trivalent du Groupe 3b anhydres peuvent être aisément préparés en formant les complexes nitrate des nitrates des métaux trivalents du Groupe 3b dans des conditions non-anhydres suivies par un séchage des complexes hydratés à des températures suffisantes pour déshydrater, mais insuffisantes pour décomposer de manière substantielle les complexes (moins de 5 %). Les complexes hydratés peuvent être préparés simplement en mélangeant des solutions aqueuses de nitrates des métaux du Groupe 3b trivalents avec une solution de nitrate de métal alcalin ou de nitrate d'ammonium.

Par métal alcalin, on entend le lithium, le potassium et, de préférence, le sodium. L'utilisation du nitrate de sodium dans la formation des complexes fournit un procédé avantageux quand on utilise un sel d'un sodium du composé alcoxy organique, dans la préparation des dérivés alcoxy. La réaction résulte dans la formation du nitrate de sodium en tant que précipité tel que mis en évidence par l'équation suivante pour le cérium :

$Ce(NO_3)_3,2NaNO_3$ + 3NaOX → $Ce(OX)_3$ + $5NaNO_3$

Le NaOX peut être directement combiné avec le complexe du métal du Groupe 3b trivalent ou cérium ou peut être formé in situ en combinant l'alcool, le silanol ou l'alcanolamine avec un métal alcalin ou de l'ammoniac, en présence du complexe nitrate dudit métal. Le sel de sodium (ou d'un autre métal alcalin) de l'alcool, du silanol ou de l'alcanolamine peut être utilisé comme base en vue de la réaction avec le nitrate. Cependant, le produit initial peut échanger des ligands si la réaction est effectuée en présence d'un autre alcool, silanol ou alcanolamine. Le nitrate de sodium formé en tant que sous-produit de la réaction alcoxy peut être recyclé afin d'être utilisé dans la formation des complexes, évitant ainsi la perte d'ammoniac qui est un sous-produit formé lors de la mise en oeuvre du nitrate d'ammonium.

Par métaux trivalents du Groupe 3b, on entend le scandium, l'yttrium et les lanthanides. Actuellement les actinides ne sont pas considérés comme efficaces et on peut les considérer exclus. On a discuté ci-dessus la formation du nitrate céreux par réduction du CAN.

Les complexes nitrate de métal trivalent du Groupe 3b -nitrate d'ammonium sont faciles à préparer par simple mélange dans des conditions non-anhydres. Si l'on met en oeuvre un solvant aqueux, il est préférable d'évaporer le solvant avant de sécher sous vide. On peut mettre en oeuvre différents modes de séchage par évaporation.

Le complexe hydraté sec contenant des eaux de cristallisation (coordination) peut être déshydraté par chauffage, de préférence sous vide, afin d'accélérer le séchage. On a trouvé, d'un manière inattendue, que les complexes nitrate peuvent être déshydratés jusqu'à un état anhydre, à une température plus basse et sans décomposition, contrairement aux halogénures correspondants.

Les températures et les durées suffisantes pour éliminer la quantité d'eau désirée sand provoquer une décomposition notable des complexes nitrate-nitrate d'ammonium peuvent être mises en oeuvre en vue du séchage et peuvent être déterminées aisément par l'homme de métier. On peut agiter, éventuellement.

Pour l'homme de métier, il tombe sous le sens que l'évaporation du solvant ainsi que l'élimination des eaux de cristallisation peuvent sa faire en continu.

Le vide mis en oeuvre pendant la préparation des complexes peut aller de la pression atmosphérique au vide le plus poussé possible. Dans la pratique, on a mis en oeuvre des pressions réduites comprises entre 100 mm de Hg et 400 mm de Hg et moins. Il est préférable que la pression soit inférieure à 300 mm de Hg, et encore plus préférentiellement, inférieure à 100 mm de Hg.

On devrait mettre en oeuvre des températures suffisantes pour provoquer une évaporation ou une déshydratation rapides sans décomposition notable du complexe. On considère comme efficace les températures comprises entre environ 50° C et environ 160° C, de préférence entre environ 80° C et environ 130° C.

Le nitrate d'ammonium et/ou le nitrate de métal alcalin doit être mélangé au nitrate de métal du groupe 3b hydraté en une quantité suffisante pour permettre à la déshydratation de se produire à des températures suffisamment basses pour prévenir ou éviter une décomposition totale.
Dans la pratique, on ajoute une quantité suffisante de nitrate d'ammonium ou de métal alcalin pour déplacer l'eau qui est fortement coordinée au métal. Le ligand oxygéné bidenté des nitrates ajoutés favorise le déplacement de l'eau qui, sinon, est difficile à éliminer. On a trouvé que l'on peut, en fait, mettre en oeuvre des rapports molaires des nitrates de métaux du groupe 3b à l'ammoniac on aux nitrates de métaux alcalins compris entre 1:1 et 1:5.

Les complexes nitrate de métal trivalent du groupe 3b -nitrate d'ammonium sont hygroscopiques. Il peut être nécessaire de les utiliser immédiatement ou de les manipuler avec soin pour maintenir les conditions anhydres.

Les complexes anhydres de nitrate de métaux du groupe 3b trouvent une application particulière dans la préparation de dérivés organiques nécessitant un produit de départ anhydre. Les dérivés alcoxy décrits dans la présente demande peuvent être préparés en mettant en oeuvre les complexes, décrits ci-dessus, de nitrate de métaux du groupe 3b. On peut développer d'autres applications similaires.

Le type des dérivés alcoxy selon la présente invention se présente comme suit :

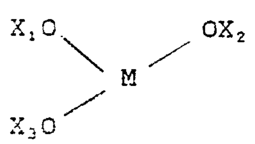

dans laquelle M est un lanthanon ou lanthanide, par exemple du cérium, $OX_1$, $OX_2$ et $OX_3$ sont choisis dans le groupe constitué par :

(a) $NO_3$
(b) OR
(c) $(O)_{4-y}$ Si $(R')_y$
(d) 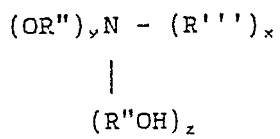

R est un radical hydrocarboné saturé ou insaturé, aliphatique ou aromatique, ayant de 1 à 20 atomes de carbone et représente, de préférence, un radical hydrocarboné linéaire, ramifié ou cycloaliphatique.

R′, identique ou différent, est un groupe hydrocarboné saturé ou insaturé ayant de 1 à 20 atomes de carbone ou un groupe alcoxy ayant de 1 à 10 atomes de carbone.
L'atome de silicium est lié à l'atome d'oxygène du groupe alcoxy. R′ peut être un groupe hydrocarboné,linéaire, ramifié, cycloaliphatique ou aromatique ou un groupe alcoxy.
De préférence, R′ a de 1 à 10 atomes de carbone et est linéaire, ramifié ou aromatique. Encore plus préférentiellement, R′ a de 1 à 6 atomes de carbone, par exemple un radical méthyle, éthyle, vinyle ou phényle.

R″,identique ou différent,est un radical hydrocarboné ayant de 1 à 20 atomes de carbone. De préférence, R″ a de 1 à 6 atomes de carbone. Encore plus préférentiellement, R″ est un radical éthyle.

R‴ est un radical hydrocarboné ayant de 1 à 20 atomes de carbone ou un atome d'hydrogène. De préférence, R‴ a de 1 à 6 atomes de carbone. Encore plus préférentiellement, R‴ est un radical éthyle, méthyle ou un atome d'hydrogène.

R, R′, R″, R‴ peuvent être substitués ou non substitués indépendamment. Aucun, un ou deux des groupes $OX_1$, $OX_2$ et $OX_3$ sont le groupe (a); les trois, deux ou un groupes $OX_1$, $OX_2$ ou $OX_3$ restants peuvent être l'un ou l'autre des groupes (b), (c) ou (d) ; et deux quelconques des groupes $X_1$ et $X_2$ et $X_3$ de la même ou d'une molécule différente de formule (I) peuvent être pris ensemble en formant un seul groupe (e), (f) ou (g) : :

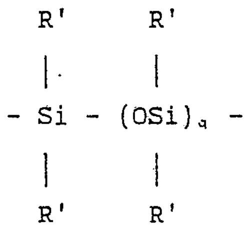 ou 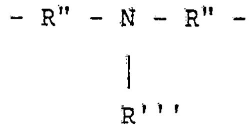 ou 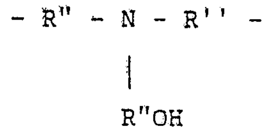

(e) (f) (g)

Si la molécule de formule (I) présente Si $(R')_y$, le nombre de groupes $Si(R')_y$ peut être alors égal à 1, 2 ou 3 et y dans (c) peut être égal à 1, 2 ou 3 (de préférence 3). Quand y = 2, le silicium de (c) est lié à deux des oxygènes tel que

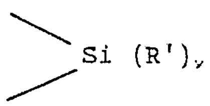

soit (1) au même atome ou (2) à un atome de cérium différent ou d'un métal trivalent du groupe 3b. Dans (1), les produits sont des monomères. Dans (2), ce sont de préférence, des oligomères ou des polymères.

Dans (d), y peut être égal à 1, 2 ou 3. z et x peuvent être, indépendamment, égaux à 0, 1 ou 2, $x + y + z$ étant égal à 3. Lorsque y = 2 ou 3, la liaison se fait avec le même atome de métal trivalent du groupe 3b (par exemple cérium) ou avec des atomes de métal trivalent différents du groupe 3b (par exemple cérium) pour former, [comme dans les groups (f) ou (g)], des oligomères ou plus vraisemblablement des polymères. q est compris entre 0 et environ 1000.

Lorsque y = 1 dans (c) ou y = 3 dans (d), le groupe (c) ou (d) est lié à n'importe lequel des 3 atomes d'oxygène des groupes $OX_1$, $OX_2$ et $OX_3$ de telle sorte qu'un seul substituant se substitue respectivement à n'importe lequel des groupes X1, X2 ou X3. Les trois atomes d'oxygène sont liés au même atome du métal du groupe 3b ou à des atomes différents du métal du groupe 3b. Ledit seul substituant est choisi parmi les suivants :

(j) 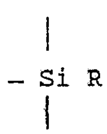 ou

(k) 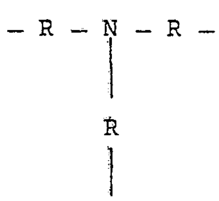

dans lesdites formules,R et R' ayant les définitions données précédemment.

Les dérivés contenant des groupes nitrato (a) sont normalement obtenus lorsque l'on ajoute le réactif alcool, silanol ou alcanolamine en quantités inférieures aux quantités stoechiométriques requises pour déplacer tous les groupes $NO_3$ du métal trivalent du groupe 3b, par exemple nitrate céreux.

Comme exemples d'alcools fournissant les groupes OR, on peut citer les alcools suivants : méthanol, éthanol, propanol, butanol, isobutanol, pentanol, isoheptanol, isopropanol, hexanol, éthyl-2-hexanol, heptanol, isoheptanol, octanol, décanol, alcool benzylique et autres alcools ayant de 1 à environ 20 atomes de carbone. Lesdits alcools peuvent être saturés ou insaturés, linéaires, ramifiés ou cycliques, aromatiques ou alkylaromatiques.

On donne des exemples de silanols correspondant à $OS(R')_y$ :

(h) 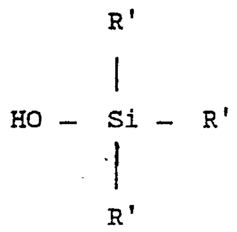

(i) 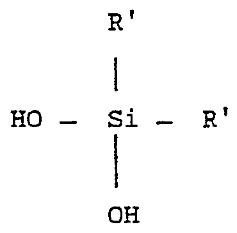

(j) 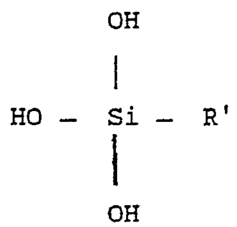 et

(k) 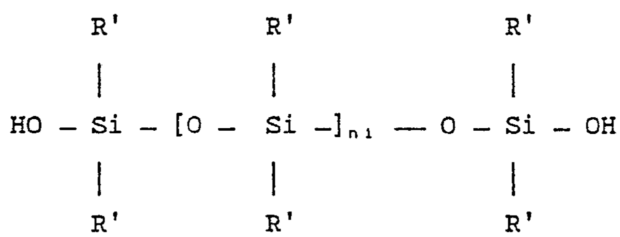

dans lesquels n1 est le nombre de ces motifs dans le polymère et peut varier de 0 à 1000, de préférence de 1 à environ 10. Le groupe (k) comprend des résines silicones solides contenant des groupes OH qui peuvent être solubilisés et utilisés dans la réaction avec des métaux trivalents du groupe 3b, par exemple, le nitrate d'ammonium céreux, pour former une résine silicone liée à l'atome de métal trivalent, par exemple céreux, par l'intermédiaire de l'oxygène. Le radical R peut être l'hydrogène ou le groupe hydrocarboné désiré dans le produit silyloxyde. Les divers groupes R′ fixés à un atome de silicium donné peuvent être, soit identiques, soit différents les uns des autres.

Les structures suivantes sont des exemples de silanols appropriés pour la présente invention : (Me représente un groupe méthyle, Ph un groupe phényle)

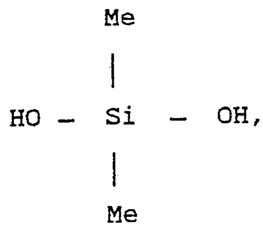 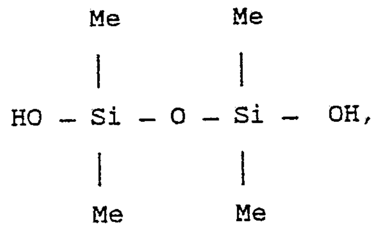

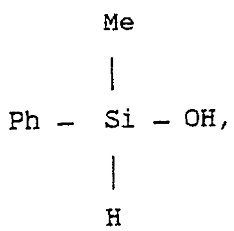 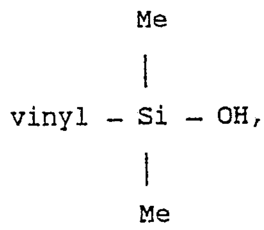 triméthylsilanol,

triéthylsilanol, triphénylsilanol, 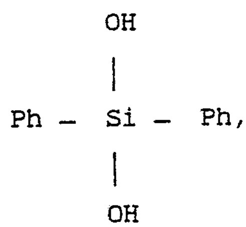

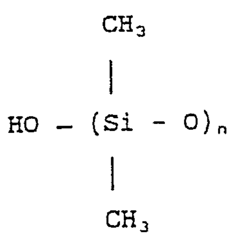 où n = 2 à 12

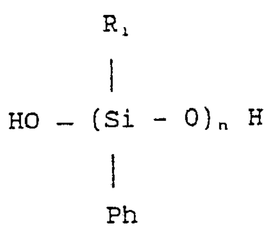 où n = 4 et $R_1$ = 1 à 6 atomes de carbone,

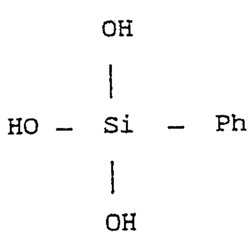 et 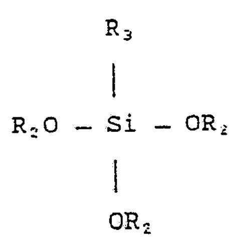

où $R_2$ = méthyle, éthyle, propyle, isopropyle ou butyle

et $R_3$ = méthyle, éthyle, vinyle ou phényle.

Pour ce qui est des silanols, conformément à la présente invention, on peut faire appel aux silanols, aux silanediols ou silanetriols ayant 2 ou 3 groupes hydrocarbonés aliphatiques tels que : triméthylsilanol, triéthylsilanol, tripropylsilanol, triisopropylsilanol, tributylsilanol, triisobutylsilanol, tri-(sec-butyl)silanol, tri-(tert-butyl)silanol, tripentylsilanol, triisopentylsilanol, tri-(sec-pentyl)silanol, tri-(tert-pentyl)silanol; diméthylsilanediol, diéthylsilanediol, dipropylsilanediol, diisopropylsilanediol, dibutylsilanediol, diisobutylsilanediol, di-(sec-butyl)silanediol, di-(tert-butyl)silanediol, dipentylsilanediol, diisopentylsilanediol, di-(sec-pentyl)silanediol, di-(tert-pentyl)silanediol; méthylsilanetriol, éthylsilanetriol, propylsilanetriol, isopropylsilanetriol, butylsilanetriol, isobutylsilanetriol, sec-butyl-silanetriol, tert-butyl-silanetriol, pentylsilanetriol, isopentylsilanetriol, sec-pentyl-silanetriol, tert-pentylsilanetriol.

Selon l'invention, on peut également mettre en oeuvre des silanols avec des groupes aliphatiques supérieurs (6 à 20 atomes de carbone), cycloaliphatiques ou aromatiques ayant de 6 à 20 atomes de carbone, ou en mélange avec des groupes aliphatiques inférieurs.

On donne, ci-après, des exemples de silanols ayant des groupes aliphatiques supérieurs, cycloaliphatiques ou aromatiques avec ou sans ligand aliphatique inférieur : trihexylsilanol, triheptylsilanol, triisoheptylsilanol, trioctylsilanol, triisooctylsilanol, tri-(éthyl-2-hexyl)-silanol, tri-(sec-octyl)-silanol, tri-(tert-octyl)silanol, trinonylsilanol, triisononylsilanol, tridécylsilanol, tridodécylsilanol, tritétradécylsilanol, trioctadécylsilanol, trihexadécylsilanol, trioléylsilanol et trieicosylsilanol ; dihexylsilanediol, diheptylsilanediol, diisoheptylsilanediol, dioctylsilanediol, diisooctylsilanediol, di-(éthyl-2-hexyl)-silanediol, di-(sec-octyl)-silanediol, di-(tert-octyl)-silanediol, dinonylsilanediol, diisononylsilanediol, di-décylsilanediol ; hexylsilanetriol, heptylsilanetriol, iso-heptylsilanetriol, octylsilanetriol, isooctylsilanetriol, 2-éthyl-hexyl-silanetriol, sec-octylsilanetriol, tert-octylsilanetriol, nonylsilanetriol, isononylsilanetriol, décylsilanetriol, ou un silanolcycolaliphatique ayant de 3 à 20 atomes de carbone, comme par exemple tricyclopropylsilanol, µtricyclobutylsilanol, tricyclopentylsilanol, tricyclohexylsilanol, tricycloheptylsilanol, tricyclooctylsilanol, tricyclododécylsilanol, tripropylcyclohexylsilanol, triméthylcyclohexylsilanol et triméthylcycloheptylsilanol ; dicyclopropylsilanediol, dicyclobutylsilanediol, dicyclopentylsilanediol, dicyclohexylsilanediol, dicycloheptylsilanediol, dicyclooctylsilanediol, dipropylcyclohexylsilanediol, diméthylcyclohexylsilanediol et diméthylcycloheptylsilanediol ; cyclopropylsilanetriol, cyclobutylsilanetriol, cyclopentylsilanetriol, cyclohexylsilanetriol, cycloheptylsilanetriol, cyclooctylsilanetriol, propylcyclohexylsilanetriol, méthylcyclohexylsilanetriol et méthylcyclohexylsilanetriol et méthylcycloheptylsilanetriol ; un silanol aromatique ou alkylaromatique ayant de 6 à 20 atomes de carbone comme par exemple triphénylsilanol, tribenzylsilanol, triphénéthylsilanol, triphénylpropylsilanol, triphényloctadécylsilanol et trinaphtyldécylsilanol ; diphénylsilanediol, dibenzylsilanediol, diphénétylsilanediol, diphénylpropylsilanediol ; phénylsilanetriol, benzylsilanetriol, phénétylsilanetriol, phénylpropylsilanetriol, naphtylsilanetriol (lorsque les triols sont trop instables, on les utilise sous la forme de leurs éthers).

Comme exemples d'alcanolamines pouvant être mises en oeuvre selon la présente invention, on peut citer : la monoéthanolamine, la diéthanolamine, la triéthanolamine, les alkylalcanolamines telles que l'éthyldiéthanolamine, la diéthyléthanolamine, etc...

L'un des procédés représentatifs de la présente invention pour préparer des composés alcoxy de cérium $^{3+}$ de formule (I) :

$$X_1O - \underset{\displaystyle OX_3}{\overset{}{\underset{|}{Ce}}} - OX_2 \qquad (I)$$

consiste à faire réagir un alcool, un silanol ou une alcanolamine appropriés avec un complexe nitrate céreux, nitrate d'ammonium, en présence d'une base,
dans laquelle,
$OX_1$, $OX_2$ et $OX_3$ sont choisis indépendamment dans le groupe formé par :

(a) $NO_3$
(b) OR
(c) $(O)_{\overline{4-y}}\ Si-(R')_y$
(d) $(OR)_y - \underset{\displaystyle (ROH)_z}{\underset{|}{N}} - R_x$

R, identique ou différent, est un groupe hydrocarboné substitué ou non ayant de 1 à 20 atomes de carbone, R′, identique ou différent, est un groupe hydrocarboné substitué ou non ayant de 1 à 20 atomes de carbone ou est un atome d'hydrogène;
dans laquelle aucun, un ou deux des groupes $OX_1$, $OX_2$ et $OX_3$ sont le groupe (a), les trois, deux ou un groupe restants,identiques ou différents, étant n'importe lequel des groupes (b), (c) ou (d) dans lesquels y est égal à 1, 2 ou 3, x et z sont égaux, indépendamment, à 0, 1 ou 2 et $x + y + z = 3$ ;
ou deux quelconques des groupes $X_1$, $X_2$ et $X_3$ sont représentés par un seul groupe ayant la formule :

```
        |
(e) - Si - R'2
 (f) - R - N - Rx où x = 1 ou
           |
           R
 (g) - R - N - (ROH)z où z = 1
           |
           R
```

Lorsque y = 2, l'atome de silicium dans (c) et l'azote dans (d) sont chacun liés à deux des atomes d'oxygène comme dans (e), (f) et (g), soit,

(1) au même atome de cérium, auquel cas les produits sont des monomères,

(2) à un atome de cérium différent, auquel cas les produits sont des oligomères ou des polymères.

De préférence, on prépare le complexe nitrate céreux, nitrate d'ammonium en réduisant le nitrate cérique d'ammonium par l'ammoniac ou un alcool.

Comme exemples de base intervenant dans le procédé ci-dessus, on peut citer l'ammoniac, le sel de métal alcalin de l'alcool ou un sel de métal alcalin du silanol.

La présente invention inclut également un composé de cérium obtenu selon le procédé ci-dessus. Le composé répond à la formule (I) suivante :

```
X1O - Ce  - OX2    (I)
      |
      OX3
```

dans laquelle :
les groupes $OX_1$ $OX_2$ et $OX_3$ sont choisis indépendamment dans le groupe formé par :

(a) $(O)_{4-y}$ Si $(R')_y$ où R′ est un groupe hydrocarboné ayant de 1 à 20 atomes de carbone ou un atome d'hydrogène,

```
(b) (OR)y - N - Rx
            |
            (ROH)z
```

où R est un groupe hydrocarboné ayant de 1 à 20 atomes de carbone, y est égal à 1, 2 ou 3, x est égal à 0, 1 ou 2 et z est égal à 0, 1 ou 2 et $x + y + z$ est égal à 3,
ou deux quelconques des groupes $X_1$, $X_2$ et $X_3$ sont représentés par un seul groupe de formule :

```
      |
(c) - Si - R'2

(d) - R - N - Rx   où x = 1 ou
          |
          R
          |

(e) - R - N - (ROH)z    où z = 1
          |
          R
          |
```

si la molécule comporte le groupe (c), le nombre de groupes (c) est de 1, 2 ou 3.

quand y = 2, l'atome de silicium dans (a) et l'azote dans (b) sont liés chacun à 2 des atomes d'oxygène de $OX_1$, $OX_2$ et $OX_3$ comme dans (c), (d), ou (e) soit :

(1) au même atome de cérium, auquel cas les produits sont des monomères,

(2) à un atome de cérium différent, auquel cas les produits sont des oligomères ou des polymères.

Les exemples suivants illustrent l'invention sans toutefois en limiter la portée.

La présence ou l'absence de $Ce^{4+}$ est déterminée en ajoutant du sulfate de baryum-diphénylamine à un échantillon acidifié par l'acide acétique glacial. En présence de $Ce^{4+}$, la solution présente une couleur pourpre intense.

Exemple 1 - Réduction du CAN par l'ammoniac dans le pentane

On met en suspension 70,5 g (0,128 mole) de CAN préalablement séché (jaune orangé) dans 500 ml de pentane. On remplace l'atmosphère de gaz inerte par de l'ammoniac et on agite bien tout le système pendant 24 h. Pendant cette durée, la matière en réaction devient incolore, ce qui indique que la réduction est terminée. On filtre la suspension et on sèche le nouveau composé sous vide poussé et on l'utilise sans autre purification pour les réactions ultérieures. Rendement : 79,4 g d'une poudre incolore. Le produit contient de l'ammoniac coordiné.

Exemple 2 - Réduction du CAN par l'ammoniac dans le diméthoxyéthane

On dissout 5 g (9,12 mmoles) de CAN dans 60 ml de DME, on dégaze la solution, on la sature par l'ammoniac et on agite pendant une nuit. Le matin suivant, le solide en suspension a complètement perdu sa couleur jaune orangé.

Exemple 3 - Réduction du CAN par l'ammoniac, sans solvant

On agite énergiquement 5 g (9,12 mmoles) de CAN sans solvant en atmosphère d'ammoniac pendant une semaine. Après cette durée, tout le CAN s'est décoloré. Le test du cérium (IV) est négatif.

Exemple 4 - Tentatives pour isoler le $Ce(NO_3)_3$ non complexé

L'extraction fractionnée par le méthanol dans lequel $NH_4NO_3$ est suffisamment soluble a échoué. Le $NH_4NO_3$ semble fortement lié au $Ce(NO_3)_3$. Cependant, l'ammoniac lié est complètement dissocié du complexe pendant le traitement, ce qui laisse le complexe $Ce(NO_3)_3.3NH_4NO_3$ de base.

Les essais pour séparer $NH_4NO_3$ par sublimation sous vide poussé à une température supérieure à 100° C se sont soldés seulement par une perte de $NH_4NO_3$.

Exemple 5 - Méthylates et isopropylates de Ce(III)

On dissout le CAN préalablement séché dans un excès de méthanol et on expose à la lumière solaire jusqu'à ce que tout le $Ce^{+4}$ soit réduit.

On traite une portion un mélange de réaction par le gaz $NH_3$, ce qui précipite $Ce(OCH_3)_3$. On sépare le méthylate du nitrate d'ammonium par extraction et lavage au méthanol, ou bien on utilise le mélange de réaction dans la synthèse ultérieure sans séparation.

On traite une autre portion en séparant sous vide tout l'excès de méthanol et l'acétaldéhyde qui est un produit d'oxydation du méthanol par le CAN. On mélange le solide restant avec l'isopropanol et ensuite on fait réagir avec l'isopropylate de sodium pour transformer $Ce(NO_3)_3$ en $Ce(OiPr)_3$.

Exemple 6 - Dérivés d'alcanolatoamines de cérium (III)

On combine dans diverses proportions des portions du complexe $Ce(NO_3)_3$-$NH_4NO_3$ agité dans le méthanol avec les alcanolamines suivantes :

monoéthanolamine (1:1, 1:2, 1:3)

diéthanolamine (1:1, 1:2)

triéthanolamine (1:1)

diéthanolamine (1:1, 1:2, 1:3)

Après l'addition d'ammoniaque, on obtient les alcanolatoamines correspondantes, en passant par l'échange avec le $Ce(OCH_3)_3$ produit in situ.

Exemple 7

On chauffe au reflux pendant une nuit 3,44 g de CAN avec 46,4 g de DME. Le jour suivant, il ne reste pas de $Ce^{+4}$ dans le ballon. Le pH résultant est de 4,0, ce qui indique que le $HNO_3$ mis en liberté a réagi avec les sous-produits d'oxydation.

Exemple 8

On mélange 5,0 g de CAN avec 50 ml de DME en présence de 0,4 g de catalyseur au charbon de bois. Tout le $Ce^{+4}$ est réduit en moins de 1,5 h et le pH final est de 1,0.

Exemple 9

On mélange en atmosphère d'argon 5,0 g de CAN avec 45,6 g de méthanol à la température ambiante en agitant en présence de 0,06 g de catalyseur platine/carbone. La réduction complète est obtenue en 30 min et le pH final est de 1,0.

Exemple 10

On agite 5,0 g de CAN, 45 g d'alcool isopropylique et 0,06 g de catalyseur à 5 % de ruthénium sur carbone à la température ambiante en atmosphère d'argon. La réduction complète de $Ce^{+4}$ est observée en 2 h.

Exemple 11

On expose à la lumière solaire pendant un jour 2,0 g de CAN mélangé avec 20 ml de méthanol. La réduction complète se produit.

Exemple 12

On répète le mode opératoire décrit à l'exemple 11, sauf que l'on remplace le méthanol par l'isopropanol, et on obtient des résultats semblables.

Exemple 13

On répète le mode opératoire décrit à l'exemple 11, sauf que l'on remplace la lumière solaire par la lumière UV, et l'on obtient des résultats semblables.

Exemple 14

On mélange 5,0 g de CAN, 44,7 g de n-butanol et 0,06 g de catalyseur à 5 % de ruthénium sur carbone. La réduction complète est obtenue en 1 h 1/2.

Exemples 15 à 39

On prépare des complexes nitrate de lanthanide-nitrate d'ammonium en mélangeant des solutions aqueuses des nitrates dont la liste figure ci-après, avec différentes quantités de nitrate d'ammonium. On évapore à sec les produits de la réaction (éventuellement en agitant afin d'obtenir un séchage uniforme) et, ensuite, on sèche sous vide dans les conditions spécifiées ci-après. On obtient les complexes nitrate de lanthanide-nitrate d'ammonium correspondants.

| Example n° | Métal | Support $M(NO_3)_3$:$NH_4$-$NO_3$ | Température séchage °C | Durée heures | Teneur en eau % en poids |
|---|---|---|---|---|---|
| Sous 135 mm de mercure | | | | | |
| 15 | La | 1:1 | 70 | 20 | Néant |
| 16 | Ce | 1:1 | 70 | 20 | 2,1 |
| 17 | Ce | 1:1 | 70 | 40 | 2,1 |
| 18 | Ce | 1:1 | 70 | 60 | 1,5 |
| 19 | Pr | 1:1 | 70 | 20 | 0,3 |
| 20 | Pr | 1:1 | 70 | 40 | Néant |
| Sous 135 mm de mercure | | | | | |
| 21 | La | 1:2 | 90 | 0 | 0,88 |
| 22 | La | 1:2 | 90 | 60 | Néant |
| 23 | Ce | 1:2 | 90 | 0 | 1,56 |
| 24 | Ce | 1:2 | 90 | 60 | Néant |
| 25 | Pr | 1:2 | 90 | 0 | 3,35 |
| 26 | Pr | 1:2 | 90 | 60 | Néant |
| 27 | Nd | 1:2 | 90 | 0 | 0,55 |
| 28 | Nd | 1:2 | 90 | 60 | Néant |
| 29 | Y | 1:1 | 110 | 48 | 15,55 |
| 30 | Y | 1:1 | 110 | 70 | 6,9 |
| 31 | Y | 1:2 | 110 | 48 | 1,08(1) |
| 32 | Y | 1:2 | 110 | 70 | 0 |
| 33 | Y | 1:3 | 110 | 48 | 1,77(1) |
| 34 | Y | 1:3 | 110 | 70 | 0 |
| Sous 135 mm de mercure | | | | | |
| 35 | La | 1:3 | 90 | 15 | Néant |
| 36 | Ce | 1:3 | 90 | 15 | Néant |
| 37 | Pr | 1:3 | 90 | 24 | Néant |
| 38 | Nd | 1:3 | 90 | 24 | Néant |
| Sous 0,01 mm de mercure | | | | | |
| 39 | Ce | 1:2 | 50 | 3 | Néant |

(1) Produit extrêmement hydrofuge, reprise d'humidité possible en cours d'analyse

A titre de contrôle, on a séché du $La(NO_3)_3$ sans nitrate d'ammonium pendant 200 heures à 95°C sous 135 mm de mercure. Le produit contient encore 5,47 % d'eau.

En revanche, un nitrate de lanthane avec du nitrate d'ammonium (proportion 1:1) présente une teneur en eau "nulle" après séchage à 70°C, pendant 20 heures seulement, sous 135 mm de mercure (voir exemple 15).

Exemple 40 - Réaction du nitrate de cérium (III) avec l'isopropylate de sodium

A une solution fraîchement préparée de 1,85 mmole du complexe de nitrate de cérium III dans le DME, on ajoute 11,1 mmoles (4,17 ml d'une solution 2,66 M) de NaOiPr dans l'isopropanol. Après 2 h, on filtre le mélange de réaction, ce qui donne 801 mg de $NaNO_3$ = 85,0 % de la quantité de $NaNO_3$ attendu (il reste un peu de solide sur les parois du verre fritté). On sèche le filtrat, on le délaie dans le pentane et on filtre. Rendement : 810 mg (1,63 mmole) : = 88,1 % pour $Ce(OiPr)_3(HOiPR)_3$ (PM = 497,67).

Le spectre de RMN est pratiquement identique à celui d'un composé préparé indépendamment par réaction de $CeCl_3$ avec NaOiPr dans le mélange THF/IPA. (tétrahydrofurane - alcool isopropylique).

Exemple 41 - Réaction d'un complexe de nitrate céreux avec le triphénylsilanol

$Ce(NO_3)_3.3NH_4NO_3 + 3Ph\ SiOH + 3NH_3 \rightarrow Ce(OSiPh_3)_3 + 6NH_4NO_3$

On met en suspension 5 g de $Ce(NO_3)_3.3NH_4NO_3$ dans 40 ml (35 g) de DME. On ajoute 7,32 g de $Ph_3SiOH$ à l'état solide et on agite pendant 5 mn. Pendant les cinq minutes suivantes, on fait barboter du gaz d'ammoniac dans la suspension, en formant une phase supérieure limpide jaune clair. On continue l'addition de $NH_3$ gazeux pendant les dix minutes suivantes. Ensuite, la filtration du mélange sur un verre fritté de Schlenk donne un filtrat jaune clair limpide. Après séparation du solvant sous le vide de la pompe à huile, on obtient 5,8 g d'une poudre blanche.

Bien soluble dans le $CHCl_3$, le DME et le THF.

Spectre de RMN de $H^1$ ($CHCl_3$-d), δ 7,24 ; 7,59

Spectre de RMN de $C^{13}$ ($CHCl_3$-d), δ 127,76 ; 129,93 ; 134,91.

Exemple 42

Réaction de $Y(NO_3)_3.4NH_4NO_3$ avec $KOC(CH_3)_3$.

On dissout 3,98 g (0,0355 mole) de tert-butoxyde de potassium dans 60 ml de THF. A cette solution limpide, presque incolore, on ajoute 3 g (0,00507 mole) de $Y(NO_3)_3.4NH_4NO_3$ sous forme solide. Dès l'agitation, il se développe une réaction exothermique et on observe la formation de $NH_3$ gazeux. On agite le mélange pendant une nuit, ensuite on filtre et on obtient 3,40 g de $KNO_3$ sous forme de poudre blanche (théorie : 3,58 g). On évapore à sec le filtrat limpide, orange pâle et on obtient une poudre ayant la couleur du tan.
Rendement : 1,4 g = 89,6 % de $Y(OC(CH_3)_3)_3$
Soluble dans la plupart des solvants organiques.

Le spectre RMN de $H^1$ ($CHCl_3$-d) présente un singulet principal à 1,21 ppm et deux signaux moins importants à 1,36 et 1,59 ppm.

Exemple 43

Réaction de $Y(NO_3)_3.4NH_4NO_3$ avec $7NaO(CH_2)_3N(CH_3)_2$.

A 2 g (3,36 mmoles) de $Y(NO_3)_3.4NH_4NO_3$ en solution dans 30 ml de THF, on ajoute 67 ml (23,5 mmoles) d'une solution 0,35 M de $NaO(CH_2)_3N(CH_3)_2$ dans du toluène. On agite la solution pendant une nuit, on filtre et on lave avec 20 ml de toluène.
On isole : 1,93 g (22,7 mmoles) de $NaNO_3$ (contenant encore 23 mg de Y sous forme de cendres).
Rendement : 1,19 g (3,01 mmoles) = 89,6 % de $Y[O(CH_2)_3N(CH_3)_2]_3$

Exemple 44

Reaction de $Y(NO_3)_3.4NH_4NO_3$ avec $7(CH_3CH_2)_2\ N(CH_2)_2\ ONa$.

A 2,17 g (3,65 mmoles) de $Y(NO_3)_3.4NH_4NO_3$ et 3,56 g (25,56 mmoles) de $NaO(CH_2)_2N(CH_2CH_3)_2$, on ajoute 50 ml de THF. On agite le mélange réactionnel durant une nuit, on le filtre et on lave le précipité trois fois avec 2-4 ml de THF. Ensuite, on élimine le solvant du filtrat.
On isole : 1,94 g (22,83 mmoles) de $NaNO_3$ = 89,3 %.
Rendement : 856 g (1,96 mmoles) de $Y[OCH_2CH_2N(CH_2CH_3)_2]_3$ = 53,7 %.

Même exemple que ci-dessus :

A 1,70 g (2,85 mmoles) de $Y(NO_3)_3.4NH_4NO_3$ et 2,78 g (19,94 mmoles) de $NaO(CH_2)_2N(CH_2CH_3)_2$ à - 78° C, on ajoute 50 ml de THF refroidi préalablement. La réaction commence en-dessous de 0° C. Elle se poursuit toute la nuit, on filtre et on lave le précipité avec du THF. On élimine se solvant.
On isole : 1,31 g (15,4 mmoles) de $NaNO_3$ - 77%.
Rendement : 1,02 g (2,3 mmoles) de $Y[OCH_2CH_2N(CH_2CH_3)_2]_3$ = 82 %.

Exemple 45

Reaction de $La(NO_3)_3.NH_4NO_3$ avec $4NaOCH_2CH_2N(CH_2CH_3)_2$.

A 1,97 g (5,06 mmoles) de $La(NO_3)_3.NH_4NO_3$ on ajoute 90 ml d'une solution 0,225 M (20,25 mmoles) de $NaOCH_2CH_2N(CH_2CH_3)_2$ dans le toluène. La réaction ne se développe pas dans le toluène que l'on élimine donc en partie (il en reste 50 ml) et on ajoute 20 ml de THF. La réaction, alors, se développe. On laisse le mélange réagir durant la nuit, on filtre, on lave le précipité et on élimine le solvant du filtrat.
On isole : 1,67 g (19,6 mmoles) de $NaNO_3$ = 97,1 % . On a retrouvé une petite quantité de produit dans $NaNO_3$ (équivalent à 35,4 mg de $Ca_2O_3$ trouvés par analyse des cendres).

Exemple 46 : Réaction du nitrate d'ammonium céreux avec le 2-diéthylaminoéthanolate de sodium dans le THF

On dissout partiellement dans 150 ml de THF 3 g (5,16 mmoles) de $Ce(NO_3)_32$, $5NH_4NO_3xDME$ (poids moléculaire = 580,86 g). A la solution, on ajoute goutte à goutte, en agitant 3,96 g (28,42 mmoles) de 2-éthylaminoéthanolate de sodium dissous dans 30 ml de THF. Il se forme immédiatement un précipité blanc avec dégagement d'ammoniac. Après l'addition des 2/3 de la solution, le mélange réactionnel devient légèrement jaune et, ensuite, il redevient lentement blanc après l'ajout de la totalité du sel. On chauffe le mélange réactionnel à environ 60° C pendant la nuit en continuant d'agiter, et on filtre. Le rendement en nitrate de sodium est de 2,3 g. On élimine le solvant du filtrat sous pression réduite à température ambiante, on obtient une huile jaune clair. Cette huile est très sensible à l'air (exposée à l'air, elle devient verte, puis brunâtre en une minute).
- Précipité :
2,3 g (27,1 mmoles) = 95 % contenant seulement des traces de cérium.
Filtrat : aucun nitrate n'est détecté
Soluble dans le méthane, le benzène, le tétrahydrofurane et le pentane.
Rendement : 96 %.

Exemple 47

Réaction du nitrate d'ammonium céreux avec le 2-éthylaminodiéthanolate de disodium dans le THF.

Dans 150 ml de THF, on dissout partiellement 2,197 g (4,51 mmoles) de $Ce(NO_3)_32NH_4NO_3$. A la solution, on ajoute lentement en agitant 2 g (11,3 mmoles) de sel de disodium dissous dans 60 ml de THF. On observe un dégagement d'ammoniac. Il se produit un précipité blanc et on agite le mélange réactionnel durant une nuit. On filtre le mélange et on obtient 1,97 g de nitrate de sodium (rendement quantitatif). On évapore le filtrat à sec et on obtient 1,945 g de poudre blanc pâle.

Teneur en cérium : 29,43 %
Rendement : 91 %
Teneur en cérium théorique : 29,54 %
Le composé est sensible à l'air, soluble dans THF, $CDCl_3$ et MeOH et insoluble dans pentane et iPrOH.

Exemple 48

Réactions du nitrate d'ammonium céreux avec les sels de lithium d'alcanolamines.

Réaction de $(NH_4)_2Ce(NO_3)_5$ avec le 2-aminoéthanolate de dilithium. On dissout partiellement dans 150 ml de THF 3 g (6,17 mmoles) de $Ce(NO_3)_32NH_4NO_3$. Dans le ballon, on ajoute goutte à goutte une solution de 2,23 g (15,43 mmoles) de sel de dilithium dans le THF. Tout est en solution après 5 minutes d'agitation. La solution passe au jaune claire avec dégagement d'ammoniac. On agite la solution toute une nuit. On élimine le solvant et on le remplace par de l'acétonitrile. On filtre le mélange réactionnel ce qui conduit à 1,4 g de $LiNO_3$ (65,8 %). On évapore à sec le filtrat restant et on obtient 2,62 g de poudre verdâtre.

Rendement : 79 %.

Exemples 49-61

Dans les exemples 49-53, on décrit la préparation des tris(triphénylsilyloxydes) de Ln(III) par réaction de $Ln(NO_3)_3\ xNH_4NO_3$ anydre avec $NaOSiPh_3$, par exemple :

$$Pr(NO_3)_3.2NH_4NO_3 + 5NaOSiPh_3 \xrightarrow{THF} Pr(OSiPh_3)_3.xTHF + NaNO_3 + 2Ph_3SiOH + 2NH_3$$

Après séparation de $NaNO_3$, par filtration, le produit précipite dans la solution de THF sous forme de dérivé du THF. Les deux moles de $Ph_3SiOH$ qui se sont formées durant la réaction, restent en solution.

Dans les exemples 54-61, on décrit la réaction des nitrates anhydres avec les sels de disodium de $Ph_2Si(OH)_2$ ou de tétraméthyldisiloxanol, par exemple :

$$Pr(NO_3)_3.2NH_4NO_3 + 2{,}5Ph_2Si(ONa)_2 \longrightarrow Pr(O_2SiPh_2)_{1.5} + Ph_2Si(OH)_2 + 5NaNO_3 + 2NH_3$$

Dans ces réactions, les produits ne précipitent pas dans les filtrats réactionnels comme c'est le cas dans les exemples 49-53. On les obtient en évaporant à sec les solutions réactionnelles. Il se produit une réaction unilatérale lorsque la mole de $Ph_2Si(OH)_2$ qui s'est formée durant la réaction, subit une condensation partielle et donne de l'eau qui, en présence de $NH_3$, hydrolyse le nitrate de lanthanide et donne du $Ln(OH)_3$ insoluble. Néanmoins, la majeure partie du complexe $Ln(NO_3)_3$ n'est pas hydrolysée et donne bien un produit Ln-O-Si.

Exemples 49-53

Procédé général pour la préparation des tris (triphénylsilyloxydes) de Ln (III)

On introduit sous forme dans un appareil Schlenk, le nitrate de métal anhydre et $NaOSiPh_3$ dans un rapport stoechiométrique et l'on charge 40 ml de THF.

Dès que l'on agite, on observe un dégagement d'ammoniax et l'on agite en continu pendant une nuit. Dans un traitement subséquent, on filtre le précipité blanc ($NaNO_3$) sur verre fritté Schlenk et on le lave avec 20 ml de THF.

On concentre les filtrats clairs combinés sous vide de la pompe à huile jusqu'à un volume de 40 ml et, stockés une nuit à -30° C, ils donnent des cristaux transparents brillants. On les isole par filtration et on les sèche doucement sous vide de la pompe à huile.

Exemple 49

$Ce(NO_3)_3$, $2NH_4NO_3$ = 3 g (0,00617 mole)

$Ph_3$ SiOna, THF = 11,42 g (0,0308 mole)

Cristaux incolores. Rendement (5 g) égal à environ 68 % pour $Ce(OSiPh_3)_3$. 3THF. Point de fusion = 145-146° C, sensible à l'air. Soluble dans $CHCl_3$, THF, DME, toluène.

Analyses :
- valeurs calculées: C = 67,04 ; H = 5,84 pour $Ce(OSiPh_3)_3$. 3THF
- valeurs trouvées: C = 67,06 ; H = 5,99

- quantité de $NaNO_3$ récupéré = 1,70 g
- quantité théorique pour $5NaNO_3$ = 1,75 g

Résultats RMN :
- RMN $H^1$ ($CHCl_3$-d) 0,66 (S, 24) ; 7,30 ; 9,55 ; (S,S,45)
- RMN $C^{13}$($CHCl_3$-d) 23,75 ; 61,07 ; 127,71 ; 128,57 ; 136,32 ; 146,51.

Exemple 50

$Pr(NO_3)_3$, $2NH_4NO_3$ : 2 g(0,00411 mole)

$Ph_3$ SiONa, 0,75THF : 7,31 g(0,0205 mole)

Cristaux vert clair. Rendement (2,5 g) égal à environ 50 % pour Pr(OSi $Ph_3)_3$, 3THF. Point de fusion = 130-132° C.

Stable à l'air, mais cristaux se dégradant à l'air.

Soluble dans toluène, $CH_3CN$, THF, $CHCl_3$, insoluble dans pentane.

Analyses :
- valeurs calculées : C = 66,99 ; H = 5,84 pour Pr(OSi $Ph_3)_3$, 3THF
- valeurs trouvées : C = 67,20 ; H = 6,24
- Quantité de $NaNO_3$ récupéré = 1,73 g.
- Quantité théorique pour $5NaNO_3$ = 1,74 g.

Résultats RMN :
- RMN $H^1$ ($CHCl_3$-d) 8,34 ; 8,71 ; 16,53(S,45) ; -7,48(S,12); -15,08(S,12)
-RMN $C^{13}$ ($CHCl_3$-d) 130,52 ; 143,54 ; 161,75 ; 29,66 ; 11,45.

Exemple 51

$Nd(NO_3)_3$, $2NH_4NO_3$ = 1,8 g (0,00367 mole)

NaOSi $Ph_3$, THF = 6,8 g (0,0183 mole).

Cristaux bleus. Rendement : (3 g) qui est égal à environ 69 % pour Nd(OSi $PH_3)_3$, 3THF. Point de fusion = 140-142 %. Stable à l'air, mais cristaux se dégradant à l'air. Soluble dans toluène, $CH_3CN$, THF, $CHCl_3$, insoluble dans pentane.

Quantité de $NaNO_3$ récupérée = 1,52 g

Quantité théorique pour $5NaNO_3$ = 1,56 g.

Analyses :
- valeurs calculées : C = 66,81 ; H = 5,82 pour Nd(OSi $Ph_3)_3$. 3THF
- valeurs trouvées : C = 66,61 ; H = 6,15

Résultats RMN :
- RMN $H^1$ ($CHCl_3$-d) 7,23 ; 8,73(m,45) ; -0,38 ; -1,07(S,24)
- RMN $C^{13}$ ($CHCl_3$-d) 127,60 ; 129,22 ; 135,62 ; 58,91 ; 22,61.

Exemple 52

$Y(NO_3)_3$, $4NH_4NO_3$ = 1,5 g (0,00253 mole)

$Ph_3$ SiONa, 0,75THF = 6,33 g (0,0177 mole)

Cristaux incolores. Rendement (2 g) égal à environ 65 % pour Y(OSi $Ph)_3$, 4THF. Point de fusion : 135-136° C.

Stable à l'air. Soluble dans toluène, THF, $CHCl_3$, insoluble dans $CH_3CN$ et pentane.

Analyses :
- valeurs calculées : C = 69,87 ; H = 6,40 pour Y(OSi $Ph_3)_3$, 4THF
- valeurs trouvées : C = 69,98 ; H = 6,48
- Quantité de $NaNO_3$ récupérée = 1,51 g
- Quantité théorique pour $7NaNO_3$ = 150 g

Résultats RMN :
- RMN $H^1$ ($CHCl_3$-d) 1,56 (S,16) ; 3,65(S,16) ; 7,22 ; 7,57(m,45)
- RMN $C^{13}$ ($CHCl_3$-d) 25,21 ; 69,04 ; 127,33 ; 128,68 ; 135,02 ; 139,63

Exemple 53

La $(NO_3)_3$, 2,5 $NaNO_3$ = 0,75 g (0,0014 mole)

$Ph_3$ SiONa, THF = 2,9 g (0,0078 mole)

Cristaux incolores. Rendement (1 g) égal à environ 60,5 % pour La (OSi $Ph_3)_3$, 3THF. Point de fusion = 95° C.

Stable à l'air. Soluble dans toluène, $CHCl_3$, DME, iPrOH, insoluble dans éthylèneglycol.
Analyses :
- valeurs calculées : C = 67,11 ; H = 5,84
- valeurs trouvées : C = 69,42 ; H = 5,83
- Quantité de $NaNO_3$ récupérée = 0,638 g
- Quantité théorique pour 5,5$NaNO_3$ = 0,665 g
Résultats RMN :
- RMN $H^1$ ($CHCl_3$-d) 1,52 ; 3,58 ; 7,22 ; 7,51(m)
- RMN $C^{13}$ ($CHCl_3$-d) 25,21 ; 68,76 ; 127,49 ; 129,11 ; 135,02 ; 138,00

Exemples 54 à 61

Procédé général pour la préparation des diphénylsilyloxydes de Ln (III) et des tétraphényldisiloxanes de Ln (III)

On introduit sous forme solide dans un appareil Schlenk, le nitrate de métal anhydre et les sels disodiques,soit de $Ph_2$ $Si(OH)_2$, soit de tétraméthyldisiloxanol, dans un rapport stoechiométrique, puis l'on charge 40 ml de THF. Dès que l'on agite, on observe un dégagement d'ammoniaque, et l'on agite en continu pendant une nuit. Dans un traitement subséquent, on filtre le précipité (essentiellement $NaNO_3$) sur verre fritté Schlenk et on le lave avec 20 ml de THF.

On évapore à sec les filtrats clairs combinés, par chauffage (à environ 40° C) sous vide de la pompe à l'huile et l'on obtient des poudres fines.

Exemple 54

$Ce(NO_3)_3$, $2NH_4NO_3$ = 2 g(0,004114 mole)

$Ph_2$ $Si(ONa)_2$, 2THF = 4,15 g (0,010 mole)

Poudre jaune pâle, sensible à l'air. Rendement (3,3 g) égal à environ 97 % de $Ce(O_2Si\ Ph_2)_{1,5}$, $Ph_2$ $Si(OH)_2$, 2THF.
Soluble dans la plupart des solvants organiques.
Point de fusion : 300° C
Résultats RMN :
- RMN $H^1$ ($CHCl_3$-d) 1,82(THF) ; 3,89(THF) ; 6,12(S) , 7,11(m), 7,51(d) , 8,74(d) ; 9,10(S) , 12,41.
- RMN $C^{13}$ ($CHCl_3$-d) 25,64(THF) ; 68,38(THF) ; 125,76 ; 126,98 ; 128,69 ; 130,07 ; 131,77 ; 134,54 ; 137,06 ; 138,44 ; 143,89.
Le spectre RMN est relativement complexe présentant un nombre élevé inhabituel de signaux pour les signaux du phényle, ce qui est dû au paramagnétisme de Ce (III).

Exemple 55

$Pr(NO_3)_3$, $2NH_4NO_3$ = 1 g (0,00205 mole)

$Ph_2$ $Si(ONa)_2$, 2THF = 2 g (0,00513 mole)

Poudre fine jaune-vert. Rendement (1,3 g) égal à environ 59 % pour $Pr(O_2Si\ Ph_2)_{1,5}$, $Ph_2$ $Si(OH)_2$, 2THF. Sensible à l'air modérement. N'a pas de point de fusion définitif, car devient à l'air modérement. N'a pas de point de fusion définitif, car devient une masse visqueuse aux environs de 120° C ; très soluble dans toluène, $CH_3CN$, THF, $CHCl_3$, insoluble dans pentane.
Résultats RMN :
- RMN $H^1$ ($CHCl_3$-d) 1,19(THF) ; 5,76 (s) ; 6,29 (t) ; 8,73 (m) ; 11,39(d).
- RMN $C^{13}$ ($CHCl_3$-d) 24,67(THF) ; 66,06(THF) ; 126,08 ; 127,27 ; 129,98 ; 130,96 ; 137,67 ; 140,06 ; 142,88.

Exemple 56

$Nd(NO_3)_3$, $2NH_4NO_3$ = 1 g (0,00203 mole)

$Ph_2$ $Si(ONa)_2$, 2THF = 2,06 g (0,005075 mole)

Poudre bleu pâle. Stable à l'air. Rendement (1,3 g) égal à environ 77,84 % pour $Nd(O_2\ Si\ Ph_2)_{1,5}$, $Ph_2$ $Si(OH)_2$, 2THF.
Point de fusion et solubilité idem à l'exemple 55.
Résultats RMN :
- RMN $H^1$ ($CHCl_3$-d) 1,54(THF) ; 3,27 ; 5,56(s,br) ; 6,34(s) ; 6,69(s) 7,88 (s) ; 9,29(s).
- RMN $C^{13}$ ($CHCl_3$-d) 25,27 ; 67,46 ; 126,78 ; 127,92 ; 128,79 ; 130,31, 132,42 ; 135,73 ; 142,11.

Exemple 57

$Y(NO_3)_3$, $4NH_4NO_3$ = 0,75 g (0,00169 mole)

$Ph_2$ $Si(ONa)_2$, 2THF = 1,75 g (0,00592 mole)

Poudre jaune, légère, brillante. Rendement (1 g) égal à environ 76,9 % pour $Y(O_2 Si Ph_2)_{1,5}$, $Ph_2$ $Si(OH)_2$, 2THF.

Point de fusion et solubilité idem à l'exemple 55.

Résultats RMN :

- RMN $H^1$ ($CHCl_3$-d) 1,57(THF) ; 3,31(THF) ; 7,13(m)
- RMN $C^{13}$ ($CHCl_3$-d) 25,32(THF) ; 67,63(THF) ; 127,22 ; 133,94 ; 134,75 ; 139,30 ; 141,20.

Dans les exemples qui suivent, on entend par TMDS, le sel disodique de tétraméthyldisiloxanol.

Exemple 58

$Ce(NO_3)_3$, $2NH_4NO_3$ = 1,48 g (0,00304 mole)

TMDS = 2,5 g (0,00762 mole)

Poudre jaune pâle,sensible à l'air. Rendement (0,75 g) égal à environ 45 % pour

$$Ce(O - Si - O - Si - O)_{1,5}, HO - Si - O - Si - OH$$

Point de fusion supérieur à 200°C (décomposition).

Soluble dans THF, modérement soluble dans toluène, $CHCl_3$. Insoluble dans pentane, $CH_3CN$, i-PrOH.

Résultats RMN

- RMN $H^1$ (THF-$d_8$) 0,1497 ;
- RMN $C^{13}$ (THF-$d_8$) 1,35.

Exemple 59

$Nd(NO_3)_3$, $2NH_4NO_3$ = 1,49 g (0,00304 mole)

TMDS = 2,5 g (0,00762 mole)

Poudre bleu pâle. Stable à l'air. Rendement (1 g) égal à environ 60 % pour

$$Nd(O - Si - O - Si - O)_{1,5} , HO - Si - O - Si - OH$$

Point de fusion supérieur à 240°C (décomposition).

Soluble dans THF, $CHCl_3$, toluène et insoluble dans pentane, $CH_3CN$ et I-PrOH.

Résultats RMN :

- RMN $H^1$ ($CHCl_3$-d) 1,81(THF) ; 3,77(THF) ; 0,09
- RMN $C^{13}$ ($CHCl_3$-d) 25,64(THF) ; 68,06(THF) ; 1,05.

Exemple 60

$Pr(NO_3)_3$, $2NH_4NO_3$ = 1,48 g (0,00305 mole)

TMDS = 2,5 g (0,00762 mole)

Poudre vert pâle, se dégradant à l'air. Rendement (1 g) égal à environ 59,5 % pour

```
          |       |                 |       |
Pr (O - Si - O - Si - O)1,5, HO - Si - O - Si - OH
          |       |                 |       |
```

Point de fusion = 140°C (décomposition)
Soluble dans THF, $CHCl_3$, $CH_3CN$, toluène
Légèrement soluble dans pentane.
Insoluble dans I-PrOH.
Résultats RMN :
- RMN $H^1$ ($CHCl_3$-d) 1,94(THF) ; 3,97(THF) ; 0,09
- RMN $C^{13}$ ($CHCl_3$-d) 25,64(THF) ; 68,11(THF) ; 0,72

Exemple 61

$Y(NO_3)_3$, $4NH_4NO_3$ = 1,42 g (0,00239 mole)

TMDS = 2,5 g (0,00839 mole)
Poudre jaune claire. Stable à l'air. Rendement (0,5 g) égal à environ 41,9 % pour

```
        |                                 |
Y (O - Si - O - Si - O)1,5, HO - Si - O - Si - OH
        |                                 |
```

Soluble dans $CHCl_3$, THF, toluène, pentane, $CH_3CN$, i-PrOH.
Résultats RMN :
- RMN $H^1$ ($CHCl_3$-d) 0,34
- RMN $C^{13}$ ($CHCl_3$-d) 0,78

Les exemples ci-dessus exemplifient et illustrent la présente invention et ne limitent en aucun cas sa portée.

**Revendications**

1. Procédé de préparation de oomposés alcoxy d'un métal trivalent du groupe 3b de formule (I) :

```
X1O - M - OX2
      |            (I)
     OX3
```

qui consiste à faire réagir un complexe anhydre d'un nitrate d'un métal trivalent du groupe 3b et un nitrate choisi parmi le nitrate d'ammonium ou un nitrate de métal alcalin avec un alcool, un silanol ou une alcanolamine en présence d'une base ou à faire réagir ledit complexe avec un sel de métal alcalin dudit alcool, silanol ou alcanolamine,
dans laquelle
M est un métal trivalent du groupe 3b,
$OX_1$, $OX_2$ et $OX_3$ sont choisis indépendamment dans le group formé par :
(a) $NO_3$,
(b) OR, dans laquelle R est un groupe hydrocarboné saturé ou insaturé ayant de 1 à 20 atomes de carbone,
(c) $(O)_{4-y}Si(R')_y$, dans laquelle R' est, identique ou différent, représente un groupe hydrocarboné saturé ou insaturé ayant de 1 à 20 atomes de carbone ou un groupe alcoxy ayant de 1 à 10 atomes de

carbone, et

```
(d) (OR")y - N - (R''')x
             |
           (R"OH)z,
```

dans laquelle R″, identique ou différent, représente un groupe hydrocarboné ayant de 1 à 20 atomes de carbone, R‴ représente un groupe hydrocarboné ayant de 1 à 20 atomes de carbone ou un atome d'hydrogène,
dans laquelle R, R′, R″ et R‴ sont indépendamment saturés ou insaturés,
y est égal 1, 2 ou 3 et x et z, indépendamment, sont égaux à 0,1 ou 2 et $x + y + z$ est égal à 3,
$OX_1$, $OX_2$ et $OX_3$ sont identiques ou différents et au moins l'un des groupes $OX_1$, $OX_2$ et $OX_3$ représentent l'un des groupes (b), (c) ou (d) ou deux des groupes $X_1$, $X_2$ et $X_3$ de la même molécule ou d'une molécule différente de formule (I) sont représentés par un seul groupe choisi parmi :

```
(e)     R'     R'
        |      |
     - Si  - (OSi)q -
        |      |
        R'     R'
(f)  - R" - N - R" -
            |
            R'''
(g)  - R" - N - R" -
            |
            R"OH
```

où q est compris entre 0 et environ 1000 et (e), (f) ou (g) sont liés, soit

(1) au même atome de métal trivalent du groupe 3b, auquel cas les produits sont des monomères ou

(2) à un atome différent de métal trivalent du groupe 3b, auquel cas les produits sont des oligomères ou des polymères.

2. Procédé selon la revendication 1, caractérisé en ce que le métal du groupe 3b est le cérium, et le complexe est un complexe nitrate céreux - nitrate d'ammonium, le complexe est obtenu en réduisant le nitrate cérique d'ammonium avec de l'ammoniac.

3. Procédé selon la revendication 1, caractérisé en ce que le métal du groupe 3b est le cérium, le complexe est un complexe nitrate céreux - nitrate d'ammonium, le complexe est obtenu en réduisant le nitrate cérique d'ammonium avec un alcool.

4. Procédé selon la revendication 1, caractérisé en ce que la base est l'ammoniac.

5. Procédé selon la revendication 1, caractérisé en ce que la base est un sel de métal alcalin de l'alcool.

6. Procédé selon la revendication 1, caractérisé en ce que la base est un sel de métal alcalin du silanol.

7. Procédé selon la revendication 1, caractérisé en ce que le complexe est anhydre, qu'il est obtenu en faisant réagir un nitrate de métal trivalent du groupe 3b avec du nitrate d'ammonium ou un nitrate de métal alcalin pour former un mélange suivi d'un séchage de celui-ci afin d'éliminer l'eau de coordination pour former le complexe anhydre.

8. Procédé selon la revendication 7, caractérisé en ce que l'on fait réagir le nitrate de métal du groupe 3b avec du nitrate d'ammonium.

9. Procédé selon la revendication 7, caractérisé en ce que l'on fait réagir le nitrate de métal du groupe 3b avec un nitrate de métal alcalin, ledit nitrate de métal alcalin étant le nitrate de sodium.

10. Procédé selon la revendication 9, caractérisé en ce que l'on fait réagir le complexe anhydre avec un sel de sodium de l'alcool, du silanol ou de l'alcanolamine, afin de former un précipité de nitrate de sodium comme sous-produit et que l'on récupère ensuite le précipité pour le recycler et obtenir une deuxième

quantité du complexe.

11. Procédé selon la revendication 7, caractérisé en ce que l'on met en oeuvre ledit nitrate d'ammonium ou nitrate de métal alcalin dans un rapport molaire audit nitrate de métal trivalent du groupe 3b compris entre environ 1:1 et environ 5:1.

12. Procédé selon la revendication 7, caractérisé en ce que l'on déshydrate sous vide ledit complexe à une température comprise entre environ 50° C et environ 160° C.

13. Procédé selon la revendication 12, caractérisé en ce que l'on déshydrate ledit complexe à une température comprise entre environ 80° C et environ 130° C.

14. Procédé selon la revendication 12, caractérisé en ce que l'on déshydrate ledit complexe sous une pression réduite inférieure à 300 mm de mercure.

15. Procédé selon la revendication 12, caractérisé en ce que l'on déshydrate ledit complexe sous une pression réduite de 100 mm de mercure.

16. Procédé selon la revendication 7, caractérisé en ce que ledit métal trivalent du groupe 3b est un lanthanide de numéro atomique comprise entre 57 et 60.

17. Procédé selon la revendication 7, caractérisé en ce que ledit métal trivalent du groupe 3b est l'yttrium.

18. Procédé selon la revendication 7, caractérisé en ce que R est un groupe hydrocarboné linéaire ou ramifié ou cycloaliphatique, R′ est un groupe hydrocarboné ayant de 1 à 6 atomes de carbone, R″ est un groupe éthyle et R‴ est un groupe méthyle, éthyle ou un atome d'hydrogène.

19. Procédé de préparation d'un complexe anhydre nitrate de métal trivalent du groupe 3b - nitrate alcalin, caractérisé en ce qu'il consiste :
- à faire réagir une solution aqueuse de métal trivalent du groupe 3b avec une solution aqueuse de nitrate de métal alcalin ou de nitrate d'ammonium en quantité suffisante pour obtenir un complexe hydraté de nitrate de métal alcalin qui est déshydratable à une température inférieure à 160° C,
- à déshydrater ledit complexe hydraté à une température inférieure à environ 160° C pendant une durée suffisante pour déshydrater le complexe sans provoquer une décomposition notable dudit complexe.

20. Procédé selon la revendication 19, caractérisé en ce que l'on fait réagir une solution de nitrate de métal trivalent du groupe 3b avec une solution de nitrate d'ammonium.

21. Procédé selon la revendication 19, caractérisé en ce que l'on fait réagir une solution de nitrate de métal trivalent du groupe 3b avec une solution de nitrate alcalin, ledit nitrate de métal alcalin étant le nitrate de sodium.

22. Procédé selon la revendication 19, caractérisé en ce que l'on met en oeuvre ledit nitrate de métal alcalin ou d'ammonium dans un rapport molaire audit nitrate de métal trivalent du groupe 3b compris entre environ 1:1 et environ 5:1.

23. Procédé selon la revendication 19, caractérisé en ce que l'on déshydrate ledit complexe à une température comprise entre environ 50° C et 160° C.

24. Procédé selon la revendication 23, caractérisé en ce que ladite température est comprise entre environ 80° C et 130° C.

25. Procédé selon la revendication 19, caractérisé en ce que l'on déshydrate ledit complexe sous une pression réduite inférieure à 300 mm de mercure.

26. Procédé selon la revendication 19, caractérisé en ce que l'on déshydrate ledit complexe sous une pression réduite inférieure à 100 mm de mercure.

27. Composé de formule (I) :

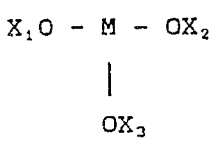

dans laquelle M est un métal trivalent du groupe 3b, $OX_1$, $OX_2$ et $OX_3$ sont choisis indépendamment dans le groupe constitué par : a) $(O)_{4-y}$ Si $(R')_y$, dans laquelle R′, identique ou différent, représente un groupe hydrocarboné ayant de 1 à 20 atomes de carbone, saturé ou insaturé, et représente un groupe hydrocarboné linéaire ou ramifié, cycloaliphatique ou aromatique ou un groupe alcoxy ayant de 1 à 10 atomes de carbone.

b) 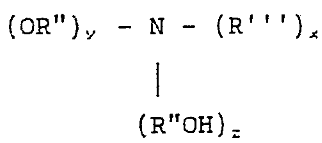

dans laquelle R″, identique ou différent, représente un groupe hydrocarboné ayant de 1 à 20 atomes de carbone, R‴ est un groupe hydrocarboné ayant de 1 à 20 atomes de carbone ou un atome d'hydrogène, y est égal à 1, 2 ou 3, x et z, indépendamment, sont égaux à 0, 1 ou 2 et x + y + z est égal à 3 ;
ou deux des groupes $X_1$, $X_2$ ou $X_3$ de la même molécule ou d'une molécule différente de formule (I) sont représentés par un seul group choisi parmi :

c) 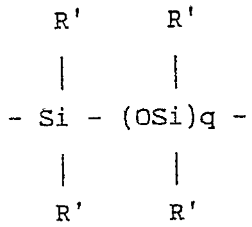

d) 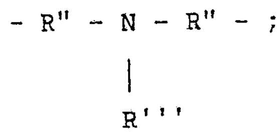 ou

e) 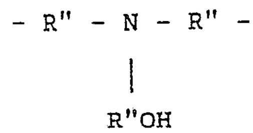

dans laquelle q est compris entre 0 et environ 1000 et (c), (d) ou (e) sont liés soit au même atome du métal du groupe 3b pour former une structure monomère ou à des atomes différents du métal du groupe 3b pour former une structure polymère ou oligomère.

28. Composé selon la revendication 27, caractérisé en ce que M est le cérium.

29. Composé selon la revendication 27, caractérisé en ce que M est l'yttrium.

30. Composé selon la revendication 27, caractérisé en ce que R′ possède de 1 à 6 atomes de carbone, R″ est un éthyle et R‴ est un éthyle, méthyle ou un atome d'hydrogène.

31. Procédé de préparation de composés alcoxy de cérium (III) de formule (I) :

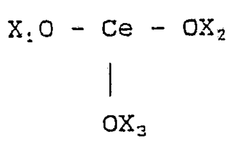

(I)

qui consiste à faire réagir un alcool, un silanol ou une alcanolamine appropriés avec un complexe nitrate céreux, nitrate d'ammonium, en présence d'une base,
dans laquelle,
$OX_1$, $OX_2$ et $OX_3$ sont choisis indépendamment dans le groupe formé par :

(a) $NO_3$

(b) OR

(c) $(O)_{4-y}$ Si $-(R')_y$

(d) $(OR)_y - N - R_x$

|

$(ROH)_z$

R, identique ou différent, est un groupe hydrocarboné substitué ou non ayant de 1 à 20 atomes de carbone,
R′, identique ou différent, est un groupe hydrocarboné substitué ou non ayant de 1 à 20 atomes de carbone ou est un atome d'hydrogène dans laquelle aucun, un ou deux des groupes $OX_1$, $OX_2$ et $OX_3$ sont le groupe (a), les trois, deux ou un groupe restants, identiques ou différents, étant n'importe lequel des groupes (b), (c) ou (d) dans lesquels y est égal à 1, 2 ou 3, x et z sont égaux indépendamment à 0, 1 ou 2 et $x + y + z = 3$ ;
ou deux quelconques des groupes $X_1$, $X_2$ et $X_3$ sont représentés par un seul groupe ayant la formule :

|

(e) - Si - R'2

(f) - R - N - Rx où x = 1 ou

|

R

|

(g) $- R - N - (ROH)_z$ où z = 1

|

R

|

Lorsque $y = 2$, l'atome de silicium dans (c) et l'azote dans (d) sont chacun liés à deux des atomes d'oxygène comme dans (e), (f) et (g), soit,

(1) au même atome de cérium, auquel cas les produits sont des monomères,

(2) à un atome de cérium différent, auquel cas les produits sont des oligomères ou des polymères.

32. Procédé selon la revendication 31, caractérisé en ce que l'on prépare le complexe nitrate céreux - nitrate d'ammonium par réduction du nitrate cérique d'ammonium avec de l'ammoniac.

33. Procédé selon la revendication 31, caractérisé en ce que l'on prépare le complexe nitrate céreux - nitrate d'ammonium par réduction du nitrate cérique d'ammonium avec un alcool.

34. Procédé selon la revendication 31, caractérisé en ce que la base est l'ammoniac.

35. Procédé selon la revendication 31, caractérisé en ce que la base est un sel de métal alcalin de l'alcool.

36. Procédé selon la revendication 31, caractérisé en ce que la base est un sel de métal alcalin du silanol.

37. Composé obtenu par le procédé selon la revendication 31, le composé répondant à la formule (I) :

$X_1O - Ce - OX_2$

|  (I)

$OX_3$

dans laquelle :
les groupes $OX_1$, $OX_2$ et $OX_3$ sont choisis indépendamment dans le groupe formé par :
(a) $(O)_{4-y}$ $Si(R')_y$ où R' est un groupe hydrocarboné ayant de 1 à 20 atomes de carbone ou un atome d'hydrogène,

(b) $(OR)_y - N - Rx$
$\qquad\qquad |$
$\qquad (ROH)_z$

où R est un groupe hydrocarboné ayant de 1 à 20 atomes
y est égal à 1, 2 ou 3, x est égal à 0, 1 ou 2 et z est égal à 0, 1 ou 2 et x + y + z est égal à 3,
ou deux quelconques des groupes $X_1$, $X_2$ et $X_3$ sont représentés par un seul groupe de formule :

$\qquad |$
(c) $- Si - R'_2$

(d) $- R - N - Rx$ où x = 1 ou
$\qquad\quad |$
$\qquad\quad R$
$\qquad\quad |$

(e) $- R - N - (ROH)_z$ où z = 1
$\qquad\quad |$
$\qquad\quad R$
$\qquad\quad |$

quand y = 2, l'atome de silicium dans (a) et l'azote dans (b) sont liés chacun à 2 des atomes d'oxygène de $OX_1$, $OX_2$ et $OX_3$ comme dans (c), (d), ou (e) soit :

(1) au même atome de cérium auquel cas les produits sont des monomères,

(2) à un atome de cérium différent, auquel cas les produits sont des oligomères ou des polymères.